# EUROPEAN PATENT SPECIFICATION

(11) **EP 3 893 974 B1**
(45) Date of publication and mention of the grant of the patent: **19.07.2023**
(21) Application number: 19797326.6
(22) Date of filing: 18.10.2019
(51) Int. Cl.: A61M 16/04

(54) **MEDICO-SURGICAL TUBE ARRANGEMENTS**
MEDIZINISCH-CHIRURGISCHE TUBUSANORDNUNGEN
ARRANGEMENTS DE TUBE MÉDICO-CHIRURGICAL

(30) Priority: 15.11.2018 GB 201818696
(43) Date of publication of application: 20.10.2021
(73) Proprietor: Smiths Medical International Limited, Ashford, Kent TN25 4BF (GB)
(72) Inventor: TUPPER, Steven Mark, Hythe, Kent CT21 5XW (GB); MCCORD, Christopher Geoffrey, Croydon, CR0 6EW (GB); PAGAN, Eric, Hythe, Kent CT21 6BD (GB); KHASAWNEH, Mohammad Qassim Mohammad, Canterbury, Kent CT1 2FP (GB); BURCHELL, Robert James, Baldock, Hertfordshire, SG7 6DA (GB); FLINT, Jonathan McNeil, Ealing, London, W5 5JD (GB)
(74) Representative: Flint, Jonathan McNeill
(86) International application number: PCT/GB2019/000148
(87) International publication number: WO 2020/099813

(56) References cited:
- WO-A1-2016/083782
- WO-A1-2018/185448
- US-A1- 2011 030 694
- US-A1- 2013 281 885
- US-A1- 2016 157 868
- US-B2- 8 473 033

## Description

This invention relates to medico-surgical tube arrangements of the kind including a medico-surgical tube having a shaft and an expansible member on the outside of the shaft adapted to make contact with the surface of a body cavity within which the tube is inserted, the tube also including a plurality of sensors on the expansible member around the expansible member responsive to contact between the expansible member and the wall of the body cavity.

Tracheal tubes are used to supply ventilation and anaesthetic gases to a patient, such as during surgery. The tracheal tube may be inserted via the mouth or nose, in the case of an endotracheal tube, or may be inserted via a surgically-made tracheostomy opening in the neck, in the case of a tracheostomy tube. Most, but not all, tracheal tubes have some form of a seal on their outside which forms a seal between the outside of the tube and the inside of the trachea so that gas flow is confined to the bore of the tube and cannot flow around the outside of the tube, between the tube and the trachea.

The most common form of seal is provided by an inflatable cuff that is inflated and deflated via a small bore lumen extending along the tube and connected towards its rear end to an inflation line terminated by an inflation indicator, valve and connector. These inflatable cuffs may be of the high-volume/low-pressure kind where the cuff is formed of a flexible plastics material moulded with a natural annular or doughnut shape that is inflated without stretching, to contact the wall of the trachea, by relatively low-pressure gas supplied via the inflation line. Alternatively, the cuff may be of the low-volume/high-pressure kind where the cuff is of an elastic material that lies close to the tube shaft when uninflated but is inflated and stretched to a larger diameter by relatively high pressure gas supplied via the inflation line. Various problems exist with both forms of cuff. One problem is the difficulty of preventing secretions that collect above the cuff leaking between the cuff and the trachea and entering the bronchial passages. The leakage of such secretions is thought to contribute to ventilator-associated pneumonia (VAP).

In order to minimise secretions leaking past the cuff it is important to maintain the optimum contact pressure of the cuff with the tracheal wall, which is typically about 25cmH₂O. If the cuff pressure is too low then it may allow passages to form along the outside of the cuff through which secretions can flow. It may also prevent effective ventilation by allowing some of the ventilation gas to escape. If cuff pressure is too high it may damage the delicate lining of the trachea. The pressure of the cuff can change during use. The gas by which the cuff is inflated may permeate through the cuff wall over time leading to a gradual loss of pressure. Alternatively, anaesthetic gas can permeate into the cuff leading to an increase in pressure. Conventional arrangements for measuring cuff pressure rely on some form of pressure sensor connected to the cuff pressure inflation line outside the body, such as described in US9180268, US20140261442, US9433737 and WO2012122267. The relatively small bore of these inflation lines means that there is a significant drop in pressure along the line so the pressure monitored is different from the actual pressure within the cuff. There is also the risk that any kink in the inflation line or any external pressure on the inflation indicator balloon connected to the inflation line could affect the pressure reading. US2011030694 describes a cuffed endotracheal tube with a sensor to determine proper placement of the tube. US2013281885 describes an endotracheal tube with sensors on its cuff and a display that indicates correct placement of the tube. WO2018185448 describes a cuffed tube with an RFID sensor on the cuff to indicate its pressure.

Another problem with conventional sealing cuffs is that there is no way of determining whether there is a localised region around the cuff where there is incomplete contact that might allow leakage of gas and secretions between the cuff and the tracheal wall and to warn of such leakage or potential leakage. The irregular nature of the anatomy in the trachea makes it difficult to achieve a complete seal around the entire circumference without applying an excessively high pressure. To ensure a complete seal the user may overinflate the sealing cuff and cause damage to the tracheal wall.

The pressure applied by the outside of the cuff to the tracheal wall can also change if, for example, the tube is displaced or the patient's neck or head is moved.

Sealing members other than inflatable cuffs are used on some tubes, such as of an expansible foam. There are similar problems in other tubes having sealing members. Expansible members are also provided on tubes for purposes other than sealing, such as where pressure needs to be applied to tissue, such as to expand a vessel.

It is an object of the present invention to provide an alternative medico-surgical tube.

According to the present invention there is provided a medico-surgical tube arrangement of the above-specified kind, characterised in that the arrangement includes a leakage indicator unit responsive to an output from each sensor, and that the leakage indicator unit detects from the output from each sensor regions of incomplete contact between the expansible member and the wall of the body cavity and thereby provides an indication of potential leakage.

The sensor means may include a plurality of pressure sensors responsive to pressure between the expansible member and the wall of the body cavity. Alternatively, the sensor means may include electrical contacts around the expansible member. The electrical contacts may be arranged to sense an electrical property at the contacts caused by contact with the body tissue. The contacts may be arranged to detect electrical properties between different contacts around the expansible member caused by contact with body tissue. Alternatively, each sensor may include a temperature sensor. The expansible member is preferably an inflatable cuff. The tube is preferably a tracheal tube.

A tracheostomy tube and an arrangement including a tube and leakage indicator according to the present disclosure will now be described, by way of example, with reference to the accompanying drawings, in which:
- Figure 1: is a side elevation view of a tracheostomy arrangement;
- Figure 2: is an enlarged side elevation view of the region of the sealing cuff in Figure 1;
- Figure 3: is an enlarged view of the cuff of an alternative tube; and
- Figure 4: is an enlarged side elevation view of a tube for a tracheostomy arrangemen according to the present invention.

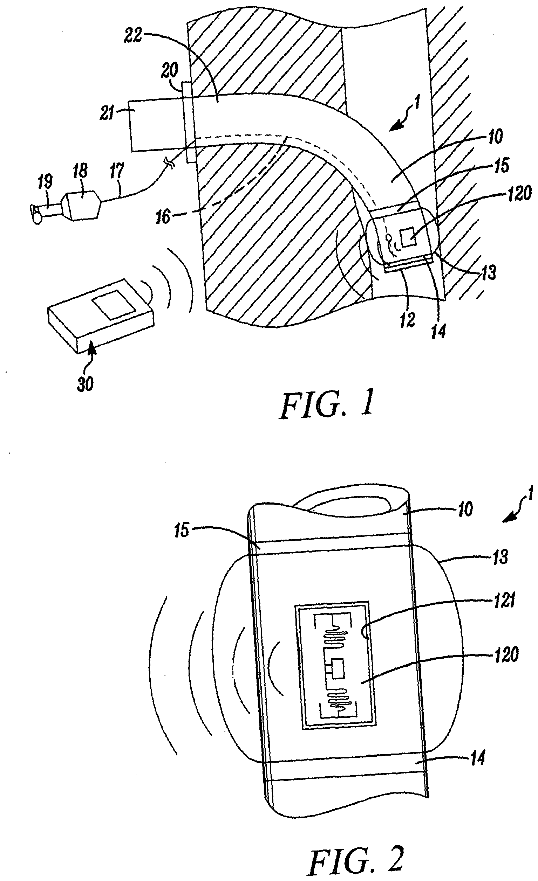

With reference first to Figures 1 and 2, the tracheostomy tube 1 comprises a curved shaft 10 of circular section with a patient end 12 adapted to be located within the trachea of the patient. The shaft 10 has an expansible member provided by a conventional inflatable sealing cuff 13 towards its patient end 12. The cuff 13 is of a thin, flexible plastics material and is attached with the shaft at opposite ends by respective collars 14 and 15. The cuff 13 may be of the low-pressure/ high-volume kind where the cuff is of a relatively floppy material that can be inflated to seal with the trachea at low pressure, typically around 25cmH₂O. Alternatively, the cuff may be of an elastic material that, in its natural state, lies close to the outside of the shaft. Such a cuff is stretched and inflated by supplying a relatively high pressure to the cuff by air or a liquid, such as saline. The cuff 13 is inflated and deflated by means of an inflation lumen 16 extending along the shaft 10 within its wall. Towards its machine end the inflation lumen 16 connects with a small bore inflation line 17 terminated by an inflation indicator balloon 18 and a combined connector and valve 19. A syringe or the like can be connected to the connector 19 to enable air or other inflation fluid to be supplied to or from the cuff 13.

The shaft 10 is moulded or extruded and is bendable but relatively stiff, being made of a plastics material such as PVC or silicone. Alternative shafts could be of a metal such as stainless steel or silver. The machine end 22 of the shaft 10 is adapted, during use, to be located externally and adjacent the tracheostomy opening formed in the patient's neck. The machine end 22 of the shaft 10 has a neck flange 20, by which the tube is secured to the patient's neck, and a coupling 21 to which a mating connector (not shown) can be connected. The mating connector is attached to breathing tubing extending to a ventilator or anaesthetic machine. Alternatively, the coupling 21 may be left open where the patient is breathing spontaneously.

The tube 1 differs from conventional tubes by the inclusion of signal means in the form of a pressure-responsive device provided by a pressure sensor 120 located in or on the cuff 13.

As shown in Figure 2 the pressure sensor is provided by a radio frequency identification (RFID) tag 120 of the kind incorporating an element responsive to pressure such as a strain gauge on a deformable substrate. This is a passive device that does not require any internal power supply but instead relies on energisation from an external source in a leakage indicator unit 30 including an RFID reader located outside the patient. In the arrangement shown in Figures 1 and 2 the RFID tag 120 is mounted on the outside of the shaft 10, preferably in a shallow recess 121 on the surface of the shaft so that the tag does not project substantially above the outer surface of the shaft. The tag 120 may be attached to the shaft 10 by an adhesive or solvent or by some mechanical means such as by staking. The tag 120 could be encapsulated within the material of the shaft providing the pressure sensing element of the tag was sufficiently exposed, such as by being covered by only a thin, flexible layer of material. The pressure sensing element could be of any conventional kind such as resistive, capacitive or inductive. The tag 120 is located beneath the cuff 13 and between the two collars 14 and 15.

When the RFID tag 120 is energised by the unit 30 it generates a radio frequency signal indicative of the pressure to which it is exposed, that is, the pressure within the cuff 13. By checking the unit 30 the clinician can confirm that the actual pressure within the cuff is within the optimum desired range. If there is any deviation from this the clinician can simply increase inflation of the cuff or open the valve 19 to allow some of the inflation fluid to escape, as appropriate. The unit 30 could be of the kind that the nurse carries around with him as he checks intubated patients. Alternatively, the unit could be mounted at the bedside and be arranged to monitor the pressure continuously or at regular intervals and to generate an alarm signal if pressure deviates from the desired range such as to indicate potential leakage between the cuff and patient tissue. The unit could be incorporated into other equipment, such as, for example a capnograph, ventilation monitor or a general vital signs monitor.

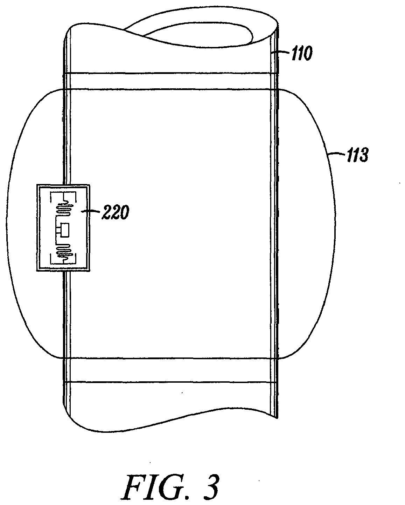

Instead of mounting the sensor on the shaft beneath the cuff it could be mounted on the cuff itself, as shown in Figure 3. This arrangement has an RFID sensor 220 that is flexible and of rectangular shape mounted aligned with the axis of the tube 110 midway along the length of the cuff 113. The sensor 220 could be of the same kind as described above, which is responsive to pressure, and in this case would respond to the contact pressure between the outside of the cuff 113 and the tracheal surface. Alternatively, the sensor 220 could provide an indication of pressure indirectly by means of a strain gauge element incorporated into the sensor that responds to flexing of the cuff 113 and sensor as the cuff is inflated and expands. Such a sensor may be more suitable for use on high-pressure cuffs of an elastic material that stretch and expand as they are inflated. Such sensors could be mounted on the outside or inside of the cuff. Where the sealing member is not an inflatable member, but is instead, for example, a foam member that expands to contact the inside of the trachea or other body cavity, the sensor is preferably mounted on the outside of the member.

The indication of pressure within the cuff need not be provided by a direct pressure measurement but could, for example, derived indirectly such as by measuring the extent of expansion of the cuff. This could be achieved by use of an RFID tag incorporating a proximity detector responsive to change in the distance between the tag and the cuff wall.

Although there are advantages to the sensor being on or in the sealing cuff it would be possible for it to be mounted at a different location in pressure communication with the cuff, such as outside the patient and in the inflation indicator balloon or connector. Although such an arrangement would not provide a direct indication of pressure in the sealing cuff it would still have the advantage of being of low cost, not requiring any internal power supply and not requiring any cable connection to the sensor.

The RFID sensors 120 and 220 could be prefabricated discrete devices mounted on or inside the cuff. Alternatively, the sensors could be printed on the shaft or the cuff, such as by laser printing with a conductive ink or by laser activation of a printed conductive ink. Alternatively, the sensor could be formed by photo lithographic techniques, or by removing material from a layer to form the desired circuit by chemical etching, laser ablation or the like.

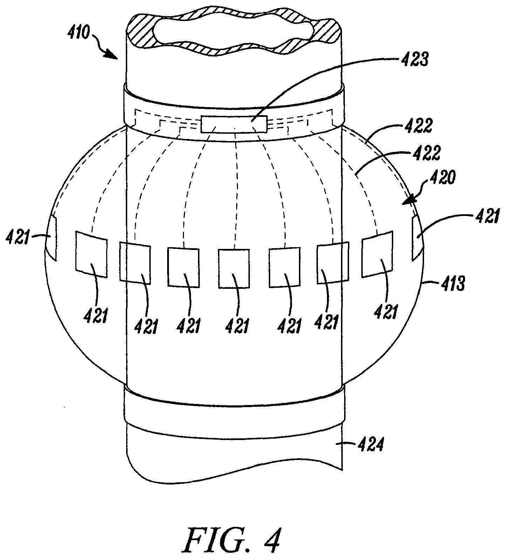

Figure 4 shows a part of a tracheostomy tube 410, according to the present invention, having an expansible sealing cuff 413. This tube 410 includes sensor means, indicated generally as 420, responsive to contact of the sealing cuff 413 with the tracheal wall around the circumference of the sealing cuff. In particular, the means responsive to contact includes a plurality of sensors 421 disposed around the cuff 413 along the part of the cuff that has the largest circumference when inflated, that is, along the annular region of the cuff that will contact the tracheal wall. There are various forms of sensors 421 that could be used for this. For example, the sensors 421 could be pressure sensors each responsive to the local pressure exerted by the cuff 413 on the tracheal wall in the region of that sensor. A low pressure output from one or more sensor 421 would indicate a poor seal in the region of that sensor and provide warning of potential leakage to indicate that it might be necessary to increase pressure within the cuff until the output from that sensor rises sufficiently to indicate a complete seal. Alternatively, the sensors 421 could be electrical contacts arranged to sense an electrical property at the contact caused by contact with an adjacent region of the tracheal wall. For example, the contacts 421 could be connected in a circuit such that the resistance, inductance or capacitance of the contact changes on contact with the tracheal wall, thereby indicating whether or not that sensor was in close contact with the tracheal wall. Any sensor 421 giving an output indicative of poor contact with the tracheal wall would indicate poor contact of the cuff 413 in that region and prompt the clinician to increase cuff pressure. Alternatively, adjacent contacts 421 could be linked in a circuit to measure electrical resistance between the contacts. A low resistance reading would be indicative of close contact with the tracheal wall whereas a high resistance would be indicative of incomplete contact of one or both contacts with the tracheal wall and indicate potential leakage. In another arrangement the sensors 421 could be temperature sensors providing individual outputs of temperature around the sealing cuff 413. Regions of close contact of the cuff 413 with the wall of the trachea would be indicated by a relatively high temperature close to body temperature whereas low temperature outputs would indicate that the cuff in the region of those sensors 421 was not making an effective contact with the wall of the trachea and hence potential leakage.

The sensors or contacts 421 provide means responsive to contact with body tissue. They may each be separate RFID tags printed on the outside of the cuff or attached to it. Alternatively, as shown in Figure 4, they are electrically connected by printed conductive tracks 422 to a common RFID tag 423 attached to the shaft 424 of the tube. The RFID tag or tags would be interrogated remotely by a reader unit held up to the neck during intubation. Other forms of means responsive to contact with body tissue could be used.

The invention is not confined to tracheal tubes but could, for example, be used in laryngeal tubes, where the preferred inflation pressure range is around 80cmH2O, or endobronchial tubes with a pressure range of 30-60cmH2O. The invention could be used on other tubes with sealing cuffs, such as foley catheters. The invention is not confined to tubes adapted to seal with surrounding tissue but could, for example, be used with tubes adapted to apply pressure to surrounding tissue, such as, for example, dilatation catheters for expanding blood vessels.

The present invention enables an indication of irregular contact of an expansible member with surrounding tissue to be provided to the user indicative of potential leakage so that inflation can be increased to improve contact. The invention also helps avoid overinflation by providing confirmation of effective contact when this has been achieved.

## Claims

1. A medico-surgical tube arrangement including a medico-surgical tube (1) having a shaft (10) and an expansible member (413) on the outside of the shaft (10) adapted to make contact with the surface of a body cavity within which the tube is inserted, the tube (1) also including a plurality of sensors (420, 421) on the expansible member (413) around the expansible member responsive to contact between the expansible member and the wall of the body cavity, **characterised in that** the arrangement includes a leakage indicator unit (30) responsive to an output from each sensor (420, 421), and that the leakage indicator unit (30) detects from the output from each sensor regions of incomplete contact between the expansible member (413) and the wall of the body cavity and thereby provides an indication of potential leakage.

2. An arrangement according to Claim 1, **characterised in that** the plurality of sensors includes a plurality of pressure sensors (421) responsive to pressure between the expansible member (413) and the wall of the body cavity.

3. An arrangement according to Claim 1, **characterised in that** the plurality of sensors includes electrical contacts around the expansible member.

4. An arrangement according to Claim 3, **characterised in that** the electrical contacts are arranged to sense an electrical property at the contacts caused by contact with the body tissue.

5. An arrangement according to Claim 3, **characterised in that** the contacts are arranged to detect electrical properties between different contacts around the expansible member caused by contact with body tissue.

6. An arrangement according to Claim 1, **characterised in that** each sensor includes a temperature sensor.

7. An arrangement according to any one of the preceding claims, **characterised in that** expansible member is an inflatable cuff (413).

8. An arrangement according to any one of the preceding claims, **characterised in that** tube is a tracheal tube (1).

## Patentansprüche

1. Medizinisch-chirurgische Schlauchanordnung mit einem medizinisch-chirurgischen Schlauch (1), der einen Schaft (10) und ein ausdehnbares Element (413) an der Außenseite des Schafts (10) aufweist, das so beschaffen ist, dass es mit der Oberfläche eines Körperhohlraums, in den der Schlauch eingeführt wird, in Kontakt kommt, wobei der Schlauch (1) auch eine Vielzahl von Sensoren (420, 421) auf dem ausdehnbaren Element (413) um das ausdehnbare Element herum aufweist, die auf den Kontakt zwischen dem ausdehnbaren Element und der Wand des Körperhohlraums ansprechen, **dadurch gekennzeichnet, dass** die Anordnung eine Leckindikatoreinheit (30) enthält, die auf einen Ausgang von jedem Sensor (420, 421) anspricht, und dass die Leckindikatoreinheit (30) aus dem Ausgang von jedem Sensor Bereiche eines unvollständigen Kontakts zwischen dem dehnbaren Element (413) und der Wand des Körperhohlraums erkennt und dadurch einen Hinweis auf ein mögliches Leck liefert.

2. Anordnung nach Anspruch 1, **dadurch gekennzeichnet, dass** die Vielzahl von Sensoren eine Vielzahl von Drucksensoren (421) umfasst, die auf den Druck zwischen dem ausdehnbaren Element (413) und der Wand des Körperhohlraums reagieren.

3. Anordnung nach Anspruch 1, **dadurch gekennzeichnet, dass** die Vielzahl von Sensoren elektrische Kontakte um das ausdehnbare Element herum umfasst.

4. Anordnung nach Anspruch 3, **dadurch gekennzeichnet, dass** die elektrischen Kontakte so angeordnet sind, dass sie eine elektrische Eigenschaft an den Kontakten erfassen, die durch den Kontakt mit dem Körpergewebe verursacht wird.

5. Anordnung nach Anspruch 3, **dadurch gekennzeichnet, dass** die Kontakte so angeordnet sind, dass sie elektrische Eigenschaften zwischen verschiedenen Kontakten um das dehnbare Element herum erfassen, die durch den Kontakt mit Körpergewebe verursacht werden.

6. Anordnung nach Anspruch 1, **dadurch gekennzeichnet, dass** jeder Sensor einen Temperatursensor enthält.

7. Anordnung nach einem der vorhergehenden Ansprüche, **dadurch gekennzeichnet, dass** das dehnbare Element eine aufblasbare Manschette (413) ist.

8. Anordnung nach einem der vorhergehenden Ansprüche, **dadurch gekennzeichnet, dass** der Schlauch ein Trachealtubus (1) ist.

## Revendications

1. Agencement de tube médico-chirurgical comprenant un tube médico-chirurgical (1) ayant une tige (10) et un élément expansible (413) sur l'extérieur de la tige (10) adapté pour entrer en contact avec la surface d'une cavité corporelle dans laquelle le tube est inséré, le tube (1) comprenant également une pluralité de capteurs (420, 421) sur l'élément expansible (413) autour de l'élément expansible sensibles au contact entre l'élément expansible et la paroi de la cavité corporelle, **caractérisé en ce que** l'agencement comprend une unité d'indicateur de fuite (30) sensible à une sortie de chaque capteur (420, 421), et **en ce que** l'unité d'indicateur de fuite (30) détecte à partir de la sortie de chaque capteur des zones de contact incomplet entre l'élément expansible (413) et la paroi de la cavité corporelle et fournit ainsi une indication de fuite potentielle.9

2. Agencement selon la revendication 1, **caractérisé en ce que** la pluralité de capteurs comprend une pluralité de capteurs de pression (421) sensibles à la pression entre l'élément expansible (413) et la paroi de la cavité corporelle.

3. Agencement selon la revendication 1, **caractérisé en ce que** la pluralité de capteurs comprend des contacts électriques autour de l'élément expansible.

4. Agencement selon la revendication 3, **caractérisé en ce que** les contacts électriques sont agencés pour détecter une propriété électrique au niveau des contacts provoquée par le contact avec le tissu corporel.

5. Agencement selon la revendication 3, **caractérisé en ce que** les contacts sont agencés pour détecter des propriétés électriques entre différents contacts autour de l'élément expansible provoquées par le contact avec le tissu corporel.

6. Agencement selon la revendication 1, **caractérisé en ce que** chaque capteur comprend un capteur de température.

7. Agencement selon l'une quelconque des revendications précédentes, **caractérisé en ce que** l'élément expansible est une manchette gonflable (413).

8. Agencement selon l'une quelconque des revendications précédentes, **caractérisé en ce que** le tube est un tube trachéal (1).
